# EUROPEAN PATENT SPECIFICATION

(11) **EP 0 746 273 B1**
(45) Date of publication and mention of the grant of the patent: **11.02.2004**
(21) Application number: 95901792.2
(22) Date of filing: 02.11.1994
(51) Int. Cl.: A61F 2/30, A61C 8/00, A61L 27/00, B23K 26/06

(54) **CONTROL OF CELL GROWTH**
ZELLENWUCHSKONTROLLE
REGULATION DE LA CROISSANCE CELLULAIRE

(30) Priority: 02.11.1993 US 146790
(43) Date of publication of application: 11.12.1996
(73) Proprietor: HOSPITAL FOR JOINT DISEASES, New York New York 10003 (US); Naiman, Charles S., Brookline, MA 02146 (US)
(72) Inventor: NAIMAN, Charles, S., Brookline, MA 02146 (US); RICCI, John, North Middletown, NJ 07734 (US); ALEXANDER, Harold, Short Hills, NJ 07078 (US)
(74) Representative: Lorenz, Werner, Dr.-Ing.
(86) International application number: PCT/US1994/012759
(87) International publication number: WO 1995/012369

(56) References cited:
- EP-A- 0 561 263
- WO-A-92/21302
- FR-A- 2 610 512
- FR-A- 2 674 744
- US-A- 3 808 606
- US-A- 4 330 891
- US-A- 4 553 272
- US-A- 4 752 294
- US-A- 4 846 835
- US-A- 4 865 608
- US-A- 5 004 475
- US-A- 5 152 791
- US-A- 5 201 766

## Description

### FIELD OF THE INVENTION

The present invention relates to an implant system for control of cell growth after surgical insertion into a bone or tissue of a patient according to the preamble of claim 1.

An implant system according to the preamble of claim 1 is known from B. Chehroudi, et al, J. Biomed. Mater. Res., 24, 1203-1219 (1990).

In many areas of the body, especially in the attachment of orthopedic implants, it is very important that certain cells be made to grow in certain specific areas and directions and similarly in other types of cells in other areas e.g. in certain areas it is desirable to have bone cell growth, while in other areas, it is desirable to have soft tissue cell growth and in still other areas it is desirable to suppress cell growth causing little or no cell growth.

Smooth implant surfaces promote the formation of thick fibrosis tissue encapsulation and rough implant surfaces promote the formation of thinner, soft tissue encapsulation and more intimate bone integration. Smooth and porous titanium implant surfaces have been shown to have different effects on the orientation of fibrous tissue cells in vitro. In addition, surface roughness was demonstrated to be a factor in tissue integration into implants having hydroxyapatite surfaces and or alter the dynamics of cell attachment and growth on polymer implants whose surfaces had been roughened by hydrolytic etching. J. M. Spivak, et al, J. Biomed. Mater. Res, 24,11211149 (1990); J. L. Ricci, et al,"Modulation of Bone Ingrowth by Surface Chemistry and Roughness", The Bone-Biomaterial Interface, University of Toronto press, Toronto, Ont., Can., 334-349 (1991); T. Albrektsson, et al, Acta. Orthop. Scand., 52, 155-170 (1981); T. Albrektsson, et al, Biomaterials, 6,97-101 (1985); T. Albrektsson, et al, Biomaterials, 7,201-205 (1986); K. A. Thomas, et al. J. biomed. Mater. Res., 19,875-901 (1985); K. A. Thomas, et al, J. Biomed. Mater. Res., 21,1395-1414 (1987); T. Inoue, et al, J. Biomed. Mater. Res., 21,107126 (1987); U. M. Gross, et al, Trans. Soc., Biomater., 13,83 (1990); B. R. McAuslan, et al., J. Biomed Maters, Res., 21,921935 (1987)].

From the examination of in vitro growth characteristics of normal cells cultured on flat surfaces there has evolved the following cell "behavioral" characteristics:
- attachment dependent growth; i.e. the dependence of normal diploid cells or substrate attachment for normal growth;
- density-dependent inhibition; i.e., the tendency of such cells to slow or stop growing once a confluent monolayer is formed;
   substrate-exploring function i.e., the ability of some types of cells to migrate on a surface in search of acceptable areas for attachment and growth; and
   contact guidance; i.e., the ability of some types of cells to migrate and orient along physical structures.

[J. L. Ricci, et al, Trans. Soc. Biomat., 17,253 (1991); J. L. Ricci, et al, Tissue-inducing Biomaterials, Mat. Res. Soc. Symp. Proc, 252,221-229 (1992); J. Ricci, et al., Bull. Hosp Joint. Dis. Orthop Inst., supra; J. L. Ricci, et al. J. Biomed Mater Res., 25 (5), supra; M. Abercrombie, et al, Exp. Cell. Res., 6, 293-306 (1954); M. Abercrombie, Proc. Roy Soc., 207B, 129-147 (1980); D. M. Brunette, et al, J. Dent. Res., 62, 1045-1048 (1983); D. M. Brunette, Exp. Cell Res., 164,11-26 (1986); P. Clark, et al, Development, 108,635-644 (1990)].

The behavioral characteristic of cellular contact guidance has been demonstrated in vitro on a variety of surfaces such as grooved titanium, grooved epoxy polymer, and collagen matrix materials of different textures and orientations.

For example, it has been shown that fibrous tissue forms strong interdigitations with relatively large grooves in the range of about 140 um can result in an effective barrier against soft tissue downgrowth perpendicular to the grooves. It has also been shown that smaller grooves on the order of about 322 um were more effective in the contact guidance of individual cells. [D. M. Brunette, et al. Development, supra; P. Clark, et al, supra.]

The findings in these publications do not address what effects different substrate microgeometrics and sizes would have on various cell colony growth and migration parameters as opposed to the morphology of individual cells. Publications do not disclose or suggest what effect different surface microgeometry of implants would have on both the rate and direction of the cell colony growth of different cells and different tissues surrounding an implant.

### SUMMARY OF THE INVENTION

This invention is directed toward microgeometric surfaces which alter the behavior of cells attached to them and that cells derived from different tissues respond differently to these surfaces.

The object of the present invention is to attain the control of the directionality of cell growth.

Different surface microgeometries were found to cause directional soft tissue and bone cell growth and control overall growth to different degrees, confirming our hypothesis that such surfaces favor one or the other type of cell growth and strongly direct that growth.

A further task of the invention is to provide a surface geometry acting as a barrier to prevent soft tissue migration into bone attachment areas.

The object as well as the further task are solved by an implant system according to claim 1.

Substrates having microgeometric texturized surfaces can be used to control the rate and orient the direction of the growth of different calls. After sufficient growth has been achieved, the particular tissue themselves can be transferred from the substrates and either implanted or used for other purposes such as cell research, growth of tissue and/or cells in bioreactors, and the like. For example, by employing micro-geometric texturized surfaces on an appropriate substrate, epidermal cells can be grown for use in repairing or replacing tissue lost from burns, nerve cells can be grown for repairing or replacing damaged nerve tissue as well as glands, bone marrow, glial cells of neurological tissue, fibroblast cells, small vessel cells, smooth muscle cells, organ cells, and the like.

### BRIEF DESCRIPTION OF THE DRAWINGS

Fig. 1 is a schematic block diagram illustrating an assemblage that can be used with the invention;
Fig. 2 is a perspective view of an optical prism that can be used with the invention;
Fig. 3 is an exaggerated elevation view of optical mirrors that can be used with the invention;
Figs. 4A-4C' and 4E-4H are diagrammatic cross sectional views of microgeometric texturized configurations that can be used on the surfaces of hip implants and dental implants;
Figs. 5A-5F are diagrammatic plan views illustrating various geometric patterns in which the microgeometric texturized configurations of Figs. 4A-4C' and 4E-4H can be arranged;
Figs. 6A-6H are also diagrammatic plan views illustrating additional geometric. patterns in which the microgeometric texturized configurations of Figs. 4A-4C' and 4E-4H can be arranged;
Figs. 7A and 7B are perspective, fragmentary views, part broken away for clarity, of microgeometric surfaces illustrating embodiments of the configurations shown in Figs. 4A-4C' and 4E-4H, 5A-5F and 6A-6H;
Fig. 8 is an elevation view of a hip implant having microgeometric texturized surfaces according to the invention;
Fig. 9 is a fragmentary, enlarged elevation view illustrating a dental implant having microgeometric texturized surfaces according to the invention;
Fig. 10 is an elevation view, part in section, illustrating a transcutaneous implant having microgeometric texturized surface according to the invention;
Figs. 11 and 12 are perspective views, part broken away to reveal details, illustrating substrates that can be used to grow and develop various types of tissue cells in vitro; and
Figs. 13-21 are graphs showing the results obtained from the Example of the invention.

### PREFERRED EMBODIMENT OF THE INVENTION

The object of the present invention are attained by a surface, a substrate, with oriented micromachined surfaces which alter the behavior of cells attached to it and which surfaces make cells derived from various tissues respond differently to the surfaces.

The structure in accordance with the present invention comprises microgrooved texture zones and barrier zones. The microgrooved textured zones with various sizes of the grooves promote different types of tissue growth. The three types of site-appropriate surface configurations which are suitable for in vivo use: (1) barrier zone surface for preventing fibrous tissue migration and/or directionally conduct fibrous tissue; (2) microgrooved textured zones having the grooves size in a specific size range to promote the bone cell growth over soft fibrous tissue growth; (3) microgrooved textured zones having the grooves sizes less approximate than a specific size range to stimulate fibrous tissue growth over the bone tissue growth.

The rate and direction of cell colony growth and the growth of different cell types can be controlled by using the system and assemblage of the invention. In general, a plurality of separate zones of texturized surfaces, each zone containing a different micro-geometric design or pattern which is presented and exposed to the particular cell type for development of its unique colony growth. These different microgeometric texturized design surfaces are provided to:
a. promote the rate and orient the direction of bone growth and discourage the growth, of soft tissue to achieve secure fixation of the implant surfaces to bone tissue;
b. promote the rate and orient the direction of the growth of soft tissue while discouraging the growth of bone tissue to achieve soft tissue integration with the implant surfaces; and
c. create a barrier that discourages the growth of soft tissue, particularly soft fibrous tissue, and thereby prevent the migration of soft tissue growth into the bone tissue attachment surfaces of the implant.

The microgeometry of the size and shape of the indentations on the surface influences the growth of certain types of cells.

Surfaces for Stimulation of Fibrous Tissue Growth. Since fibrous tissue and bone cells will be "competing" for these surfaces in vivo, the ratio of bone to soft tissue colony area increase on a given surface is an important parameter in surface selection. This ratio indicates the relative stimulation or inhibition of cell growth on these surfaces. Theoretically, this ratio would be significant to provide advantage for growth of one or the other cell type on a surface, with high ratios favoring bone cell growth and low ratios favoring fibrous tissue growth. Based on these ratios, the 2-um indentation or groove surface would provide a 32.8% decrease in bone/soft tissue growth, providing a significant advantage in soft tissue cell growth. This surface could be used to favor bone cell growth; it can also be used to significantly orient growth of these cells. The 4-um indentation or groove surface provides a similar ratio but is based on lower overall growth rates. If non-oriented fibrous cell growth is required, the flat control surface provides an inherent advantage to RTF cells a ratio of bone to soft tissue cell growth of approximately 0.6). This effect has been observed in vivo where smooth surfaces have been shown to favor formation of thick fibrous tissue capsule formation as compared to textured surfaces of the same composition, which show less fibrous capsule formation and more extensive osteointegration.

The surface with the highest ratio of bone to soft tissue cell growth is the 12-um indentation or groove substrate. The basis for the ratio is the fact that this surface inhibits soft tissue colony growth by 53.1% relative to controls. This is close to the maximal suppression of soft tissue cell growth seen on these microgeometries. This same surface, however, does not maximally suppress bone cell growth (46.3% suppression relative to controls). This differential suppression results in a 14.4% increase in bone/soft tissue growth ratio relative to the control ratio. In vivo, where both cell types are present and compete for growth, this provides a significant advantage to bone colony growth. Bone cells grown on this surface also exhibit greatly enhanced directional growth in the x-axis. In vivo, this surface should favor bone over soft tissue growth and could be used to directionally conduct bone growth.

### Some of the surface configurations are:

**Barrier Zone Surface-to Direct Conduction of Soft Tissue.** The 8-um groove, which effectively eliminates y-axis growth and stimulates x-axis growth in fibrous tissue cells, can be used as a barrier zone to prevent fibrous tissue migration and/or can be used to directionally conduct fibrous tissue.

**Enhanced Fibrous Tissue Growth Surface.** The 2-um groove can be used to stimulate fibrous tissue growth relative to bone cells. It has the additional advantage of causing specific directional migration at more than twice the rate of cells on the flat controls. In areas of an implant surface where a randomly oriented fibrous tissue layer is needed, and no directional conduction is necessary, the flat surface, which inherently favors fibrous versus bone cell growth, can be used.

**Enhanced Bone Growth Surface-to Direct Conduction of Bone Tissue.** The 12-um groove has been shown to increase the RBM/RTF cell growth ratio. This favors bone cell growth over fibrous tissue growth. In addition, this surface causes specific directional migration of bone cells at approximately twice the rate of cells on the flat surface. This surface can be used to enhance bone vs. soft tissue growth as well as direct bone growth into regions of an implant surface where bone fixation is needed.

The ability of surface microgeometry to influence cell and tissue formation-both bone and soft tissue-has already been demonstrated.

In a series of studies, RTF (rat tissue cells) and RBM (rat bone cells) cell colonies were grown on each of the six experimental and control substrates. RTF and RBM cells from the same animals were used to initiate groups of five to eight substrates per microgeometry type and cell type. Each surface had a flat area to act as an internal control. Four colonies each were initiated on the flat control and experimental sides of each 55-mm-diameter substrate.. Thus, 40-64 colonies were initiated on each type of substrate for each cell type, divided between flat control surfaces and experimental microgeometry surfaces. The colonies analyzed in these experiments were fixed and quantitated at 4 and 8 days in order to document the most consistent period in colony growth. The RTF and RBM cell colonies were measured for area, x-axis growth (parallel to surface microgeometry), and y-axis growth (perpendicular to surface microgeometry). In order to correct for small variations in initial colony size and shape, which can be affected by the way the shape of the liquid collagen drop is influenced by surface microgeometry, average colony parameters at 4 days are subtracted from the 8-day data for each cell type and surface. This yields data expressed as area, x-axis, or y-axis increase between 4 and 8 days. X-to-y-axis growth ratios and RBM cell vs. RTF cell growth ratios are also used to compare these results.

Both cell types showed consistent growth by day 4, with the RTF cells showing earlier growth than the RBM cells.

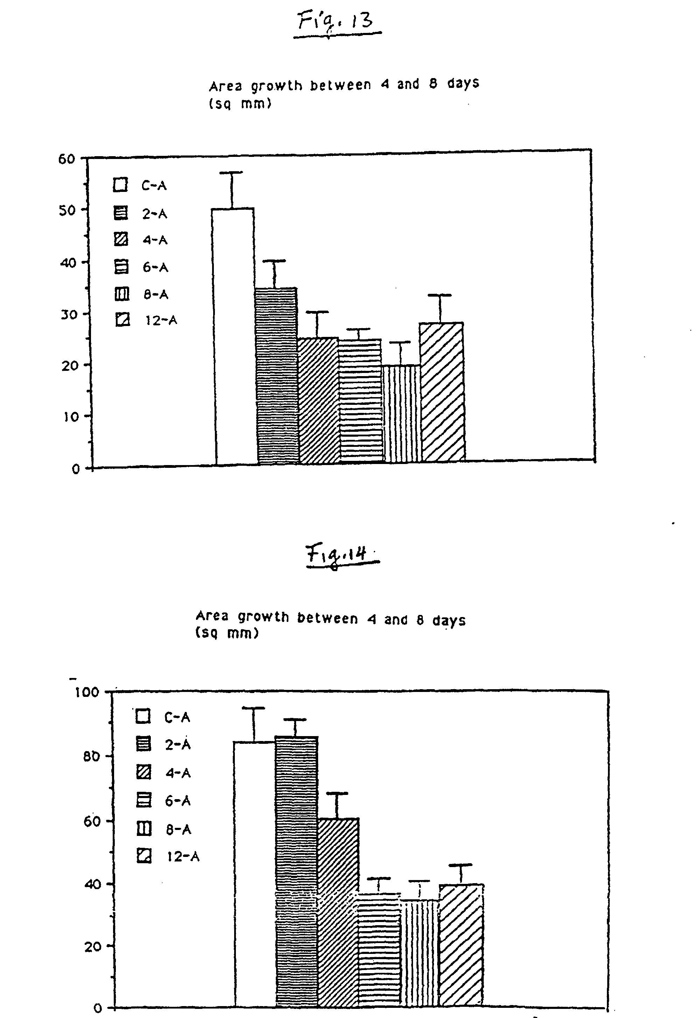

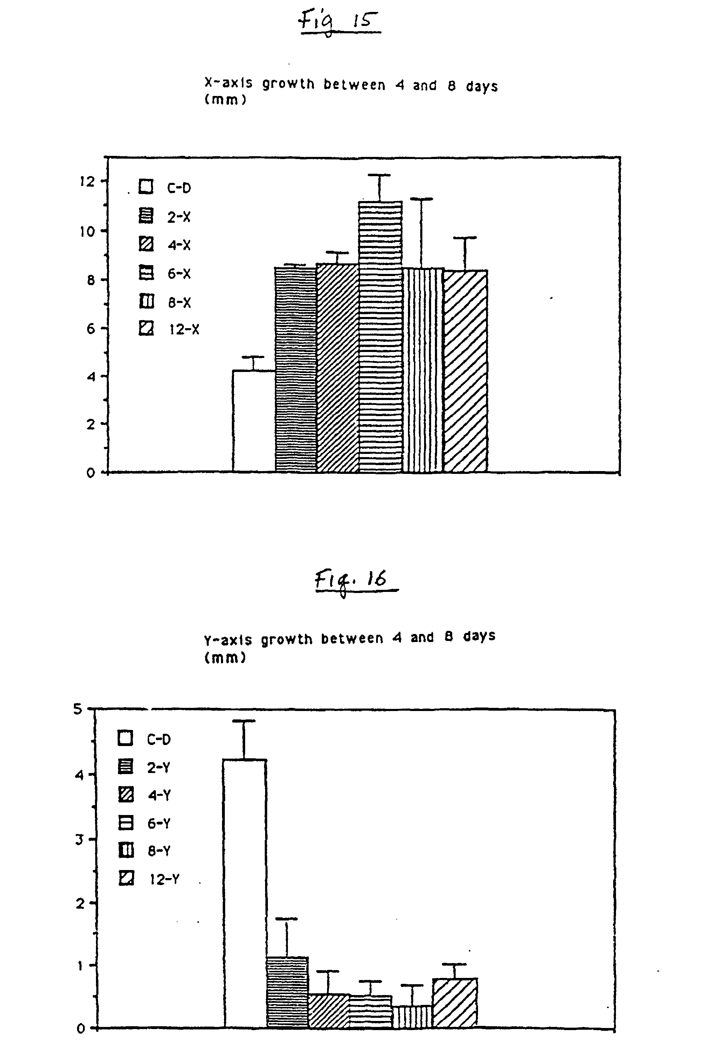

Examination of a 4-day-old RBM cell colony showed the central region, the original "dot," consisting of collagen and cells and cell outgrowth at the periphery with the cells oriented randomly and becoming sparse at the periphery. Both cells types showed extensive growth between 4 and 8 days, with the RTF cells growing at a faster rate than the RBM cells as shown in Figs. 13 and 14.

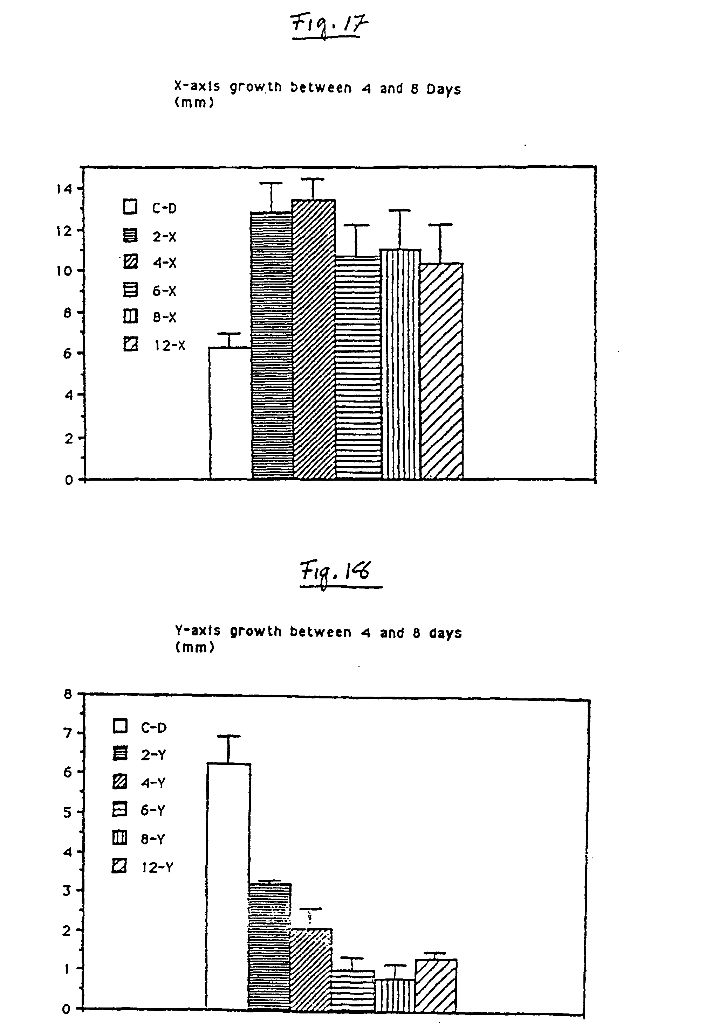

The experimental surfaces were observed to cause oriented cell attachment and migration, resulting in elongated colony growth which was accelerated in the x-direction (parallel to the surface microgeometry) and inhibited in the y-direction (perpendicular to the surface microgeometry as shown in Figs. 15-18). On an individual cell level, the cells were observed to orient along the surface grooves. This causes the cells to be "channeled" in the x-direction, as compared with control cultures, where the outgrowing cells move randomly on the flat surfaces. The most efficient cell "channeling" was observed on the 6-um and 8-um surfaces. On these surfaces, both cell types were observed to attach and orient within the grooves and on flat tops of the grooves. This resulted in enhanced x-axis growth and almost no y-axis growth by both cell types on these surfaces. On the smaller microgeometries, a different effect was observed. Both the RBM and RTF cells bridged the surfaces of the 2-um grooves resulting in cells with different morphologies than those on the 6-, 8-, and 12-um surfaces. These cells were wide and flattened and were not well oriented. On the 4-um grooves, the RBM and RTF cells showed mixed morphologies, with most cells aligned and elongated, but not fully attached within the grooves. This resulted in appreciable y-axis growth by the RBM cells on the 2-um surfaces and by the RTF cells on the 2-and 4-um surfaces. At the other end of the size range, limited y-axis growth was also observed when these cell types were grown on the 12-um surfaces. This may be a result of the microgeometry being significantly larger than the cell dimensions, allowing some diagonal cell orientation and cell "wraparound," resulting in limited y-axis growth.

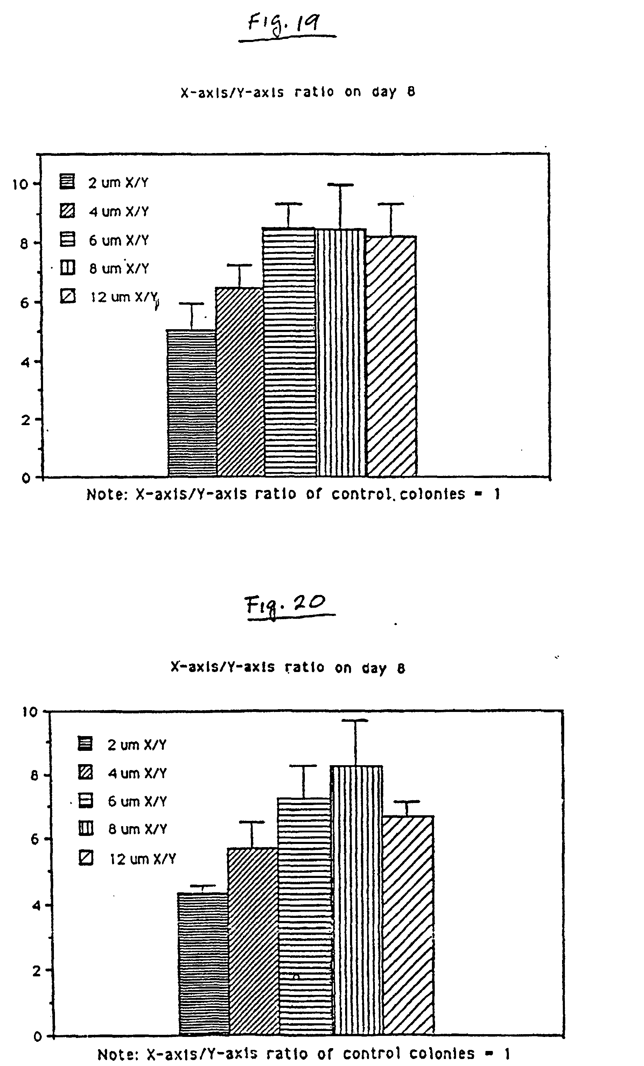

The results of the observed effects of these surfaces on overall RBM and RTF cell colony growth was very pronounced. All the experimental substrates caused varying, but significant increases in x-axis growth compared to the diameter increase of the controls and varying, but pronounces inhibition of y-axis growth (Figs. 15-18). This is nearly always resulted in suppression of overall growth of the RBM and RTF cell colonies compared with controls (Figs. 13-14). The exception was RTF cell growth on the 2-um surface (Fig. 14, which was equivalent in area increase to controls, presumably because of the y-axis growth contribution as seen in Fig. 18. Importantly, suppression of cell growth differed between cell types. This offers the opportunity to differentially provide a growth advantage to one cell type over the other.

The observed growth suppression is a geometric phenomenon. Circular control colonies increase in area as a function of the square of their radius. The colonies grown on the microgeometry surfaces become rectangular-to-oval in shape because of differential x-and y-axis growth rates. Since y-axis growth is extremely low in most cases, the colonies increase significantly in area only in the x-direction. This results in area increase based almost solely on linear increase in x-axis length. This one-dimensional growth (as compared to the two-dimensional growth of circular controls) results in a slower linear rate of area increase, even though its x-axis growth is faster than that of the control diameter growth.

This surface should prevent y-axis migration and growth and orient fibrous tissue. While the maximal orientational effect (highest x-axis/y-axis ratio) on the RBM cells was observed on the 6-, 8-, and 12-um surfaces (Fig. 19), the maximal effect on the RTF cells was observed on the 8-um surface (Fig. 20). This indicates that the best microgeometry size to act as a barner for RTF cell growth would be the 8-um surface, since RTF cells grown on this surface would have the smallest y-axis growth across this surface and an enhanced directional growth in the x-axis to values nearly twice those of controls (Figs. 18 and 20).

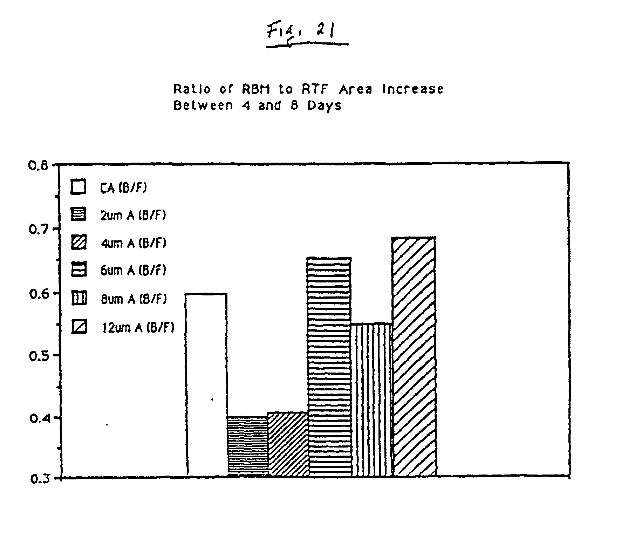

Since fibrous tissue and bone cells will be "competing" for these surfaces in vivo, the ratio of RBM to RTF colony area increase on a given surface is an important parameter in surface selection. This ratio indicates the relative stimulation or inhibition of cell growth on these surfaces (Fig. 21). Theoretically, this ratio would be significant to provide advantage for growth of one or the other cell type on a surface, with high ratios favoring bone cell growth and low ratios favoring fibrous tissue growth. Based on these ratios, the 2-um surface would provide a 32.8% decrease in RBM/RTF growth, providing a significant advantage in RTF cell growth. This surface could be used to favor RTF cell growth; it can also be used to significantly orient growth of these cells. The 4-um surface provides a similar ratio, but is based on lower overall growth rates (Figs. 13 and 14). If non-oriented fibrous cell growth is required, the flat control surface provides an inherent advantage to RTF cells (a ratio of RBM to RTF cell growth of approximately 0.6). This effect has been observed in vivo
where smooth surfaces have been shown to favor formation of thick fibrous tissue capsule formation as compared to textured surfaces of the same composition, which show less fibrous capsule formation and more extensive osteointegration.

The surface with the highest ratio of RBM to RTF cell growth is the 12-um substrate. The basis for the ratio is the fact that this surface inhibits RTF colony growth by 53.1% relative to controls (Fig. 14). This is close to the maximal suppression of RTF cell growth seen on these microgeometries. This same surface, however, does not maximally suppress RBM cell growth (46.3% suppression relative to controls, Fig. 13). This differential suppression results in a 14.4% increase in RBM/RTF growth ratio relative to the control ratio (Fig. 21). In vivo, where both cell types are present and compete for growth, this provides a significant advantage to RBM colony growth. RBM cells grown on this surface also exhibit greatly enhanced directional growth in the x-axis. In vivo, this surface should favor bone over soft tissue growth and could be used to directionally conduct bone growth.

Representative substrates that can be used to promote and develop the rate and orient the direction of the growth of various types of tissue cells in vitro are illustrated in Figs. 11 and 12. As shown in Fig. 11, one type of substrate that can be used can be provided in the form of a hollow cylindrical member 75 having opposed, opens ends 76,77, and a plurality of wedge shaped ridges 78 formed on its inner circumferential wall, the ridges 78 being parallel to each other and to the longitudinal axis of cylindrical member 75.

Another substrate embodiment is illustrated in Fig. 12 which is also in the form of a tubular member 80 having opposed open ends 81,82, and a plurality of grooves 83 formed in its inner circumferential wall 84, the grooves 83 being parallel to each other and to the longitudinal axis of tubular member 80. In this embodiment, each of the grooves 83 also has a plurality of spaced, anchoring holes 85 formed along their lengths.

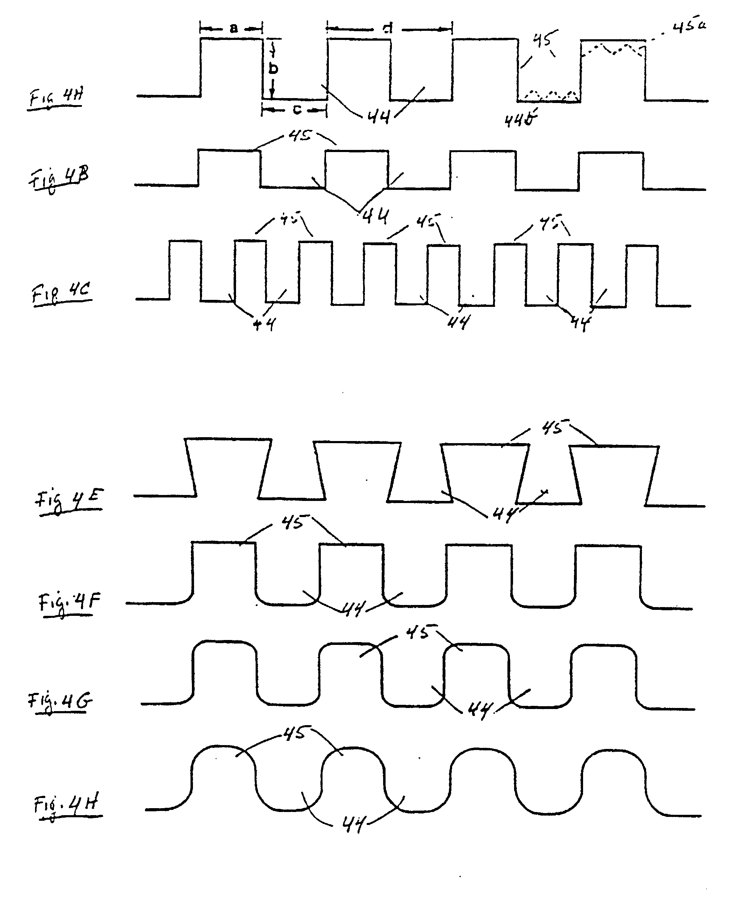

The microgeometric texturized designs and configurations on the substrates illustrated in Figs. 11 and 12 can be combined in a single substrate or the substrates can be provided with the microgeometric texturized designs and configurations illustrated in Figs. 4A-4C' and 4E-4H, 5A-5F, and 6A-6H, as well as various combinations thereof. In addition, substrates can be provided in the form of flat, planar members having one or more of these microgeometric texturized designs and configurations formed on their planar surfaces.

The efficacy of utilizing microgeometric texturized designs and configurations of the invention to promote and orient the direction of growth of tissues and cells is further illustrated by the following Example.

These studies of this Example were performed using a square wave shape (See Fig. 4A), and a flat surface as control (produced by the same methods), since the square wave shape has demonstrated a strong effect of cellular contact guidance in past studies [P. Clark, et al, Development, 108,635-644 (1990).

Following the nomenclature of Cheroudi, et al. J. Biomed. Master, Res. 24, 1067-1085 (1990)] for the surfaces, the distance comprising one groove and one ridge between the grooves is called the repeat spacing, or pitch (a+b, in Table 1). The depth (c in Table 1) of the groove refers to its deepest point, and the width (b in Table 1) is the total opening in the plane of the substrate.

The substrates used in these experiments have been molded from templates precision-fabricated at the National Nanofabrication Facility (NNF) at Cornell University. The investigators fabricated the templates with the assistance of the staff at NNF

Fabrication of the microstructures by microlithography is a three-step process: design, application of a thin photoreactive layer, and development. To model a square-wave architecture, a window with parallel grid lines was designed on the CAD program Symbad and the CAD files were used to store different grid dimensions. Vis a VAX station-3100, these files were transferred to an Optical Pattern Generator to fabricate a photomask from the design layout. This was done by exposing the window on a high-resolution emulsion plate with a laser interferometer. All photomasks were developed by typical film development procedures.

In the next step, 3-in. silicon wafers were coated with 15um of silicon dioxide, obtained by a mixture of 70% nitrous oxide and 17% silane gas, on a Chemical Deposition System. Deposition took place at a rate of 40 nm/min, with chamber conditions of 450 mtorr pressure, 50W reflecting power, and 200 C temperature. To align each grid window, the wafers were placed on a Projection Mask Aligner with their respective masks. For each template, there was a total of 239 exposures for 1.2 sec each. Exposure subsequently applied a thin layer of photoresist.

The development of square-wave microstructure was achieved by multiple reactive ion etching (RIE) techniques. Each template was initially placed on the Magnetron Ion Etcher where the silicon dixiode surface was etched with carbon tetraflouride at 1.0-kW peak voltage. Templates were next placed on the Barrel Etcher to strip the photoresist at 13.5 torr pressure and 240 C temperature. The bare polysilicon surface was further etched with the Plasma Therm RIE System. This system etches in a directional manner, producing the square-wave shape used in this example. The parameters used were 49 parts/cm3 (pccm) chlorine and 7 pccm boron trichloride, 60 mtorr pressure, and 200 kW reflecting power. Finished templates were washed in hydrochloric acid and attached to 3-in.-diameter aluminum stubs using epoxy to provide a rigid base. One-half of each template received an experimental micro-texture, the other half was left flat.

Culture plate surfaces were created from the templates by solvent casting. The mold solution was obtained by dissolving 15g of polystyrene in 100mL ethyl acetate for at least 6 hr. A dam was produced by attaching tape around the perimeter of the template. An 8-mm solvent depth produce 0.5-mm, polystyrene substrates. After pouring the solvent on the template and before it dried, they were placed under high vacuum for 1 min to eliminate air bubbles at the interface, to avoid producing inconsistencies in the experimental surfaces. The solvent was allowed to dry for 24 hr and the surfaces were placed in a water bath for 5 hrs. The substrates were peeled off their respective templates, and outer edges were removed to yield 50-mm circles.Substrates were allowed to dry for at least 24 hrs. to completely remove the solvent.

Surfaces were then evaporation-coated with titanium oxide in a Denton 502 Vacuum Evaporator. To obtain a 600-A coating, substrates were placed 100mm from the electrodes under a 40-kW reflecting power and 5 x 10-5 mtorr pressure. Substrates were ethanol-sterilized for 5 min and attached to 50-mm petri dishes with a heated probe.

To initiate RTF cell cultures, hindfoot extensor tendons were removed from male, 14-dayold Sprague-Dawley rats that had been sacrificed by C02 overdose. This method of sacrifice does not contaminate the tendon tissue. Four extensor tendons were removed under sterile conditions from each foot and placed in Hank's balanced salt solution (HBSS) containing 1% penicillinstreptomycin. The tendons were then separated from sheath tissue by microdissection. All culture procedures were conducted under sterile conditions. Cultures were grown in a 37 degree C incubator in an atmosphere consisting of 95% air, 5% C02, and saturated humidity. Cultures were maintained with Dulbecco's Modified Eagle's Medium (DMEM) containing 10% fetal bovine serum and 1% penstrep. The medium was carefully maintained at pH 7.4.

Groups of explant cultures were grown for approximately 1217 days; explant were removed, and the cultures were subcultured by enzyme digestion. Cultures were treated with a 0.25% trypsin solution in calcium-and-magnesium-free HBSS for 20 min in order to free the cells from the culture plates. Cells were rinsed in medium containing 10% serum to stop the trypsin reaction, counted using s hemocytometer, and used to inoculate culture plates or initiate dot cultures. To ensure that the cells retained their in vivo behavior characteristics, only primary, secondary, and tertiary cultures were used for the experiments.

Cell cultures were prepared from 14-day-old rat femurs according to the method of Manitopulas (Cell.Tiss.Res., 254, 317-330 (1988)). In brief, femurs were dissected out under sterile conditions and the ends cut off. The marrow cavity was flushed out with medium using a syringe and the contents were incubated in a culture plate. While the hemopoietic elements do not attach, the stromal cells attach to the plate. After the unattached elements were removed, the attached cells were allowed to grow to confluency. The cells routinely stained positive for alkaline phosphatase activity. All experiments were conducted in DMEM with 10% fetal calf serum and 1% penstrep.

When these cultures became confluent, they were subcultured using the same procedure described in"C"above for the RTF cultures.

RTF and RBM cells were embedded in a collagenous matrix as a source of cell colonies. These cultures were initiated by mixing cell suspensions with pepsin-digested type I bovine collagen solution. This solution was dispensed in 2-uL aliquots (containing 18,000-20,000 cells each) on the control and experimental substrates and polymenzed to form gels. Eight colonies were formed on each experimental substrate (designed to fit in a 60mm culture plate) with four colonies placed on the flat control side and four colonies placed on the experimental microgeometry side. The resulting gel "dots" acted as sources of cells for colony formation. The cultures were maintained under standard conditions in the appropriate medium.

Cultures were examined using an inverted-phase contrast microscope with a 35mm camera attachment.

The"dot"culture model is basically an explant model with some important distinctions. While explant cultures can be used to measure cell colony formation of surfaces, they tend to form irregular colonies whose shapes cannot be easily analyzed. This is because the explant tend to be variable in shape, cell number and area of contact with the substrate. In the present model, colony formation begins from a circular dot of cell-containing collagen gel. Colony cell numbers and shapes are consistent and colony formation kinetics are consistent and easily measured. It is the consistency and measurability of this model that makes it appropriate for these experiments.

It is believed to be important to use freshly isolated cells for experiments such as these, since continuous cell lines, transformed cell lines, and cells that have been subcultured through many passages do not exhibit attachment and migration behavior that is true to their normal in vivo state.

Cultures were preserved in 10% phosphate buffered formalin on days 4 and 8 and stained with toluidine blue stain. Representative cultures were viewed and photographed using light microscopy in order to examine individual cells as well as colony outgrowth patterns. Using the Scientific Imaging Solutions computer workstation which consists of a light microscope with a video camera connected to a personal computer with video acquisition hardware and TCL-Image image processing/image analysis software, images of the colonies were digitized and measured. Overall colony area and aspect ratios relative to the material surface microgeometry were recorded.

### NOW REFERRING TO PARTICULAR MICROGEOMETRIC PATTERNS:

The microgeometric texturized configurations illustrated in Figs. 4A-4C' and 4E-4H comprise a plurality of grooves 44 and ridges 45 formed by the system and assemblage of the invention and their dimensions are indicated by the letters "a", "b", "c" and "d". As can be seen, these configurations include those having square ridges 45 and square grooves (Fig. 4A) where "a", "b" and "c" are equal and where the spacing (or pitch) "d" between adjacent ridges 45 is twice that of "a", "b" or "c". Figs. 4B and 4C illustrate rectangular configurations formed by grooves 44 and ridges 45 where the "b" dimension is not equal to that of "a" and/or "c". Fig. 4E illustrates trapozidal configurations formed by grooves 44 and ridges 45 where the angles formed by "b" and "c" are less than 90°. In Fig. 4F, the corners formed by the intersection of dimensions "b" and "c" have been rounded. These rounded corners can range from arcs of only a few degrees to arcs where consecutive grooves 44 and ridges 45 approach the configuration of a sine curve as shown in Fig. 4H. In all of these configurations, either the planar surface of the ridge 45; i.e., the "a" dimension, or the planar surface of the groove 44; i.e., the "c" dimension, or both can be corrugated as shown by dotted lines at 45a and 44a in Fig. 4A.

In the microgeometric texturized configurations illustrated in Figs. 4A-4C' and 4E-4H, the dimension of "a" can be from about 1.5 um to about 100 um and the other dimensions, "b", "c" and "d", can be determined from the "a" dimension.

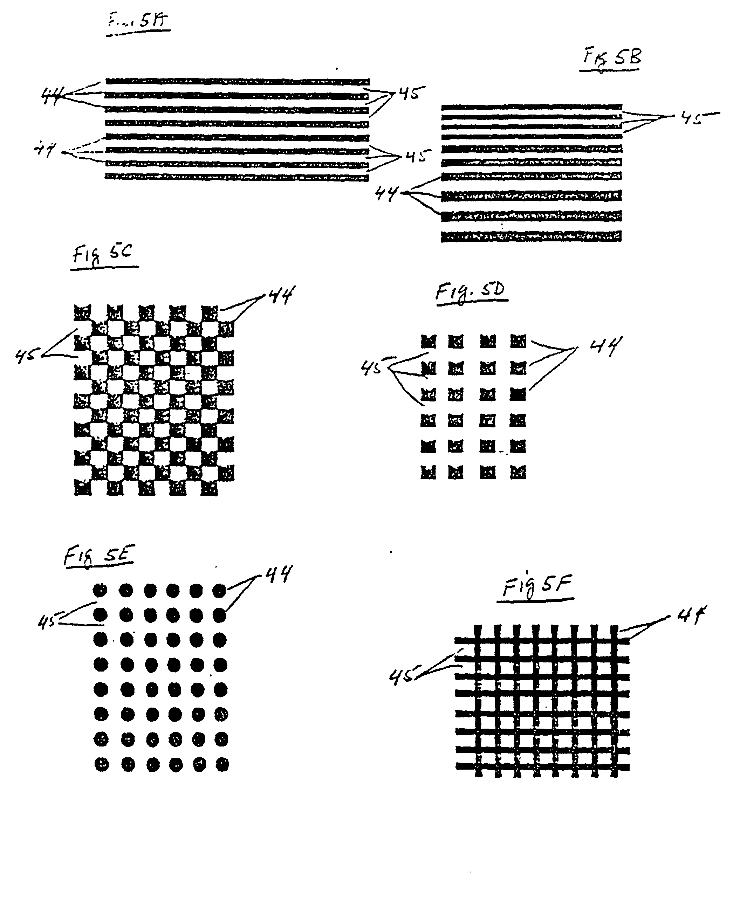

The geometric patterns in which the microgeometric texturized configurations shown in Figs. 4A-4C' and 4E-4H can be arranged are illustrated in Figs. 5A-5F. As can be seen, these unidirectional geometric patterns include those where the grooves 44 and ridges 45 are of equal size (i.e., "a"="c") and parallel (Fig. 5A); those where the grooves 44 and ridges 45 increase (or decrease) in size as shown in Fig. 5B; those where ridges 45 are in the form of individual projections defining a checkerboard configuration as illustrated in Fig. 5C; those where grooves 44 are depressed to define a plurality of spaced, parallel rectangular depressions (Fig. 5D) or a plurality of spaced, parallel circular depressions (Fig. 5E); and, those where the ridges 45 and grooves intersect one another as shown in Fig. 5F.

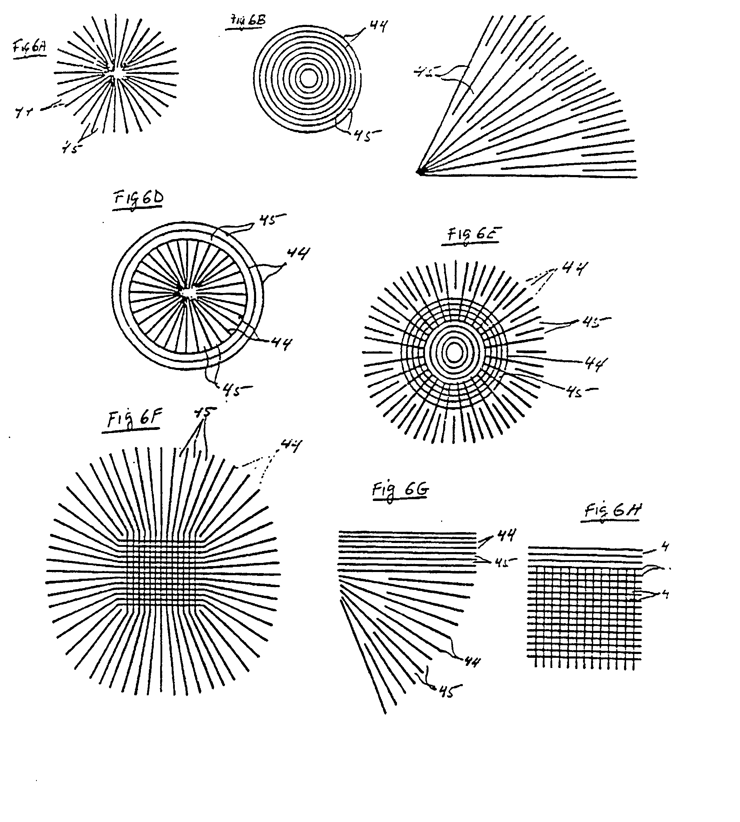

Additional geometric patterns in which the micro-geometric texturized surface configurations can be arranged are illustrated in Figs. 6A-6H in the form of unidirectional, arcuate and radial patterns, as well as, combinations thereof. As can be seen, these arrangements include radiating patterns (Fig. 6A); concentric circular patterns (Fig. 6B); radiating fan patterns (Fig. 6C); radiating/concentric circular patterns (Fig. 6D); radiating pattern intersecting concentric circular pattern (Fig. 6E); an intersecting pattern surrounded by a radiating pattern (Fig. 6F); a combination radiating fan pattern and parallel pattern (Fig. 6G); and, a combination intersecting pattern and parallel pattern (Fig. 6H).

From the embodiments illustrated in Figs. 4A-4C' and 4E-4H, Figs. 5A5F and Figs. 6A-6H, it will be appreciated that implants and substrates can be, provided with micro-geometric texturized surfaces having a multitude of geometric patterns, designs, configurations and cross sections to select from for particular implants or substrate applications.

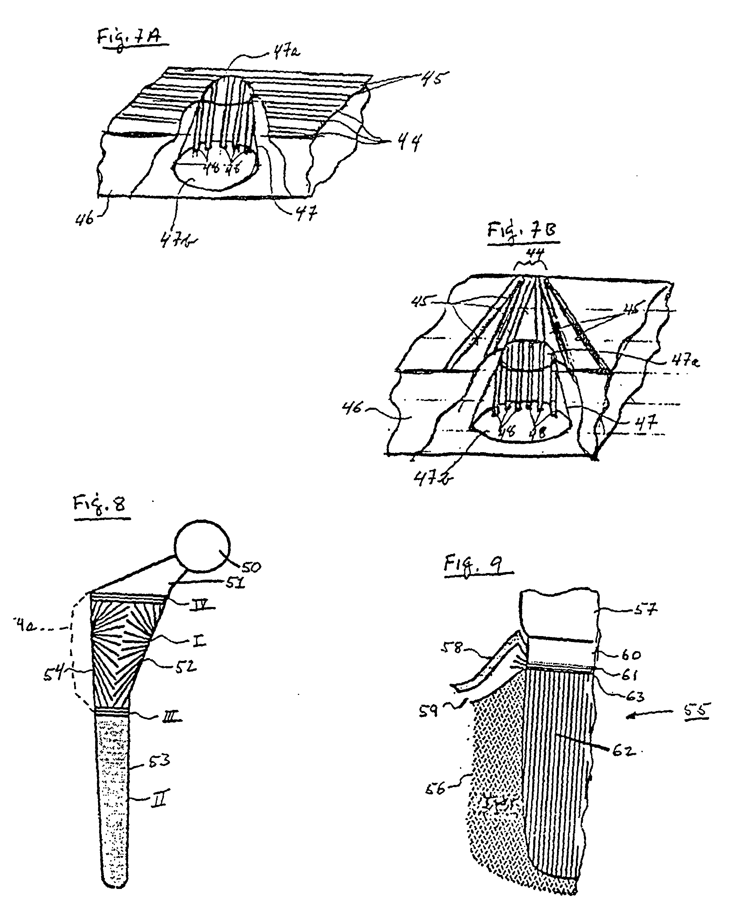

Embodiments illustrating some of these microgeometric texturized surface configurations are shown in Figs. 7A and 7B wherein grooves 44 and ridges 45 are shown formed in the surface of a typical implant 46. In addition, a cavity 47 is shown formed in the body of the implant 46, the cavity 47 having an open top 47a and a closed bottom 47b. While cavity 47 can be of any geometric configuration, it is here shown in the form of a frustoconical shape, the circumference of open top 47a being larger than the circumference of closed bottom 47b. Further, the circumferential wall of cavity 47 can have a plurality of spaced, longitudinal grooves 48 formed therein.

In the embodiment shown in Fig. 7A, the geometric pattern of the grooves 44 and ridges 45 are formed in a parallel arrangement so that grooves 44 meet the upper ends of longitudinal grooves 48 at the open top 47a of cavity 47. With this arrangement, the direction of growth of tissues and cells can be oriented not only along grooves 44 and ridges 45 on the surface of the implant 46, but along grooves 48 down into cavity 47 so that the tissues and cells become anchored in the body of the implant 46.

Although the ridges 44 and grooves 45 illustrated in the embodiment of Fig. 7B are formed in a radial arrangement, grooves 44 similarly meet the upper ends of longitudinal grooves 48 to orient the direction of growth of tissues and cells along grooves 44 and 45 and down into cavity 47 along grooves 48.

### APPLICATION OF THE INVENTION TO A HIP PROSTHESIS

A typical hip implant for humans having microgeometric texturized surfaces is illustrated in Fig. 8. As shown in Fig. 8, the implant comprises a femoral head 50, a femoral neck 51, a proximal stem 52 and a distal stem 53. Utilizing the system and assemblage of the invention, proximal stem 52 and distal stem 53 can each be ablated to create microgeometric texturized surfaces of different geometric designs or patterns in order to enhance and promote the growth of particular tissue. Premised upon the concept that oriented cells produce oriented tissue, selected portions on surface of the implant are not ablated to prevent migration of tissue growth from one part of the implant to another. Thus, four separate zones, i.e., zones I, II, III and IV, are created on the implant surface. Zone I corresponds to the texturized surface on the proximal stem 52, zone II corresponds to the texturized surface on the distal stem 53 and zones III and IV represent barrier surfaces. The texturized surface in zone I on the proximal stem 52 is patterned to promote the rate and orient the direction of the growth of bone tissue on proximal stem 52 and the texturized surface in zone II on the distal stem 53 is patterned to promote the rate and orient the direction of the growth of soft tissue on the distal stem 53. Barrier zone III is provided to prevent migration of soft tissue growth into zone I of the proximal stem 52 and prevent migration of bone tissue into zone II of the distal stem 53.. Barrier zone IV is provided to prevent migration of soft tissue from the femoral neck 51 into zone I of the proximal stem 52.

Where excessive stress is anticipated to be exerted on the lateral region 54 of the hip implant, it may be desirable to provide a reinforced hip implant where the lateral region 54 has been built up as indicated in dashed lines at 54a to impart additional strength to the implant. In either-instance, the lateral region 54 or a built up lateral region 54a is preferably not ablated to act as an additional barrier zone in preventing migration of bone tissue growth from the proximal stem 52 into either lateral region 54 or built up lateral region 54a.

Implant surfaces ablated to have individual zones of different microgeometric texturized designs or patterns separated from one another by barrier zones provide implants that have good contact in the medial and lateral regions, but not in the anterior and posterior regions; achieve bone tissue fixation in the proximal region while preventing its migration into the distal region; and, prevent the growth of fibrosis tissue into the bone tissue attachment regions.

A typical dental implant whose surfaces have been microgeometrically texturized utilizing the system and assemblage of the invention is illustrated in Fig. 9. In Fig. 9, the dental implant, generally indicated by reference numeral 55, is shown seated in bone tissue 56 with the tooth portion 57 of the implant extending below the gingival epithelium tissue 58. Between the gingival epithelium tissue 58 and the bone tissue 56 is an area of soft connective tissue 59.

The surface of dental implant 55 that comes into contact with these three different types of tissue when the implant is embedded are ablated by the system and assemblage of the invention to have different micro-geometric texturized patterns or designs for each distinct type of tissue. Thus, the surface of dental implant has a smooth zone 60 on the implant stem, a first microgeometric texturized zone 61, a second microgeometric texturized zone on the implant stem 62, and a barrier zone 63 between first and second texturized zones 61 and 62. Smooth zone 60 extends from the top of tooth 57 into and through the gingival epithelium microgeometric texturized zone 61.

Smooth zone 60 promotes the growth and attachment of gingival epithelium tissue 58 to form a seal against oral environment. This zone 60 also deters the attachment of plaque to its surface while facilitating subsequent removal of any plaque that does accumulate on it. First, microgeometric texturized zone 61 promotes the rate and orients the direction of the growth and attachment of soft connective tissue 59 and second microgeometric textured zone 62 promotes the rate and orients the direction of the growth and attachment of bone tissue 56. Barrier zone 63 is provided to prevent the migration of soft connective tissue 59 into second zone 62 and prevent the migration of bone tissue 56 into first zone 61.

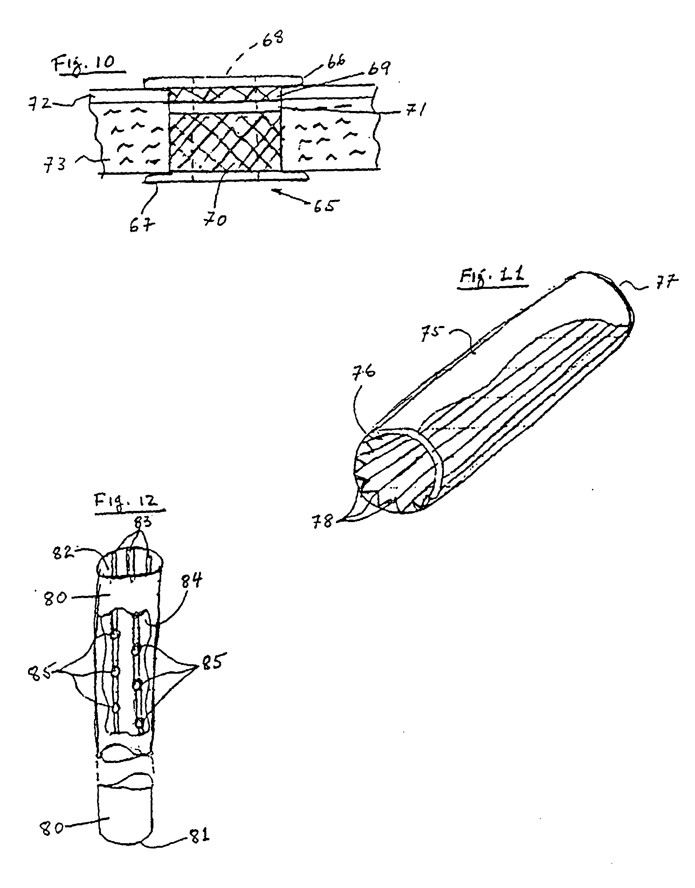

The transcutaneous implant illustrated in Fig. 10, generally identified by reference numeral 65, is typically cylindrical having upper and lower flanges 66,67 and a central longitudinal bore 68 that extends through the body of the implant 65 as indicated by the dashed lines. The outer circumferential surface of implant 65 is provided with a first microtexturized surface 69 and a second microtexturized surface 70 which are separated by a circumferential barrier zone 71.

Transcutaneous implant 65 is shown embedded in tissue consisting of epithelium tissue 72 and dermal and sub-dermal connective tissue 73. Microtexturized surface 69 promotes and orients the direction of growth of epithelium tissue 72 and microtexturized surface 70 promotes and orients the direction of growth of the dermal and sub-dermal tissue 73 into each other.

With transcutaneous implant 65 embedded in this manner, port 68 can be used as a passage to accommodate a tube for cholostomy containers or as a means to accommodate an electrode for pace makers, and the like.

To assure that the surfaces of the implants texturized by the system and apparatus of the invention are free from contaminants and debris, they can be examined using a scanning electron microscope equipped with a back scattered election imaging system and an x-ray micro-analysis system with a Quantum light element detector in conjunction with appropriate software for elemental analysis.

The texturized implants of the invention can be surgically implanted using known surgical techniques. For example, orthopaedic implants can be accomplished following the procedures described in Campbell's Operative Orthopaedics, edited by A. H. Crenshaw, published by C. V. Mosby, and dental implants can be accomplished following the procedures described in Dental Implants, edited by Charles Babbush, published by W. B. Saunders.

### A LASER SYSTEM FOR CUTTING THE GROOVES IS AS FOLLOWS:

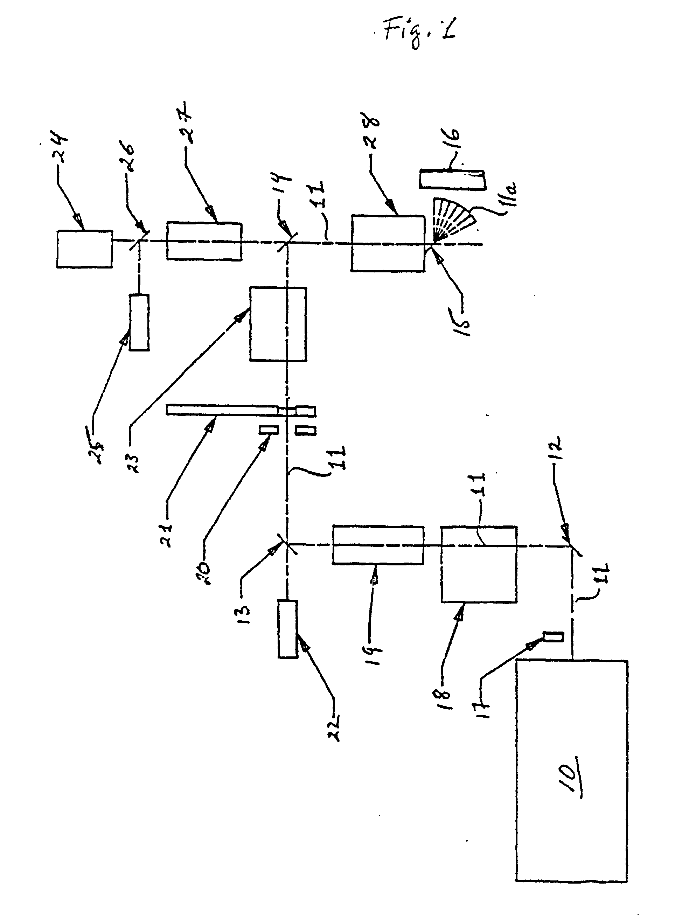

One assemblage that can be used in the system of the invention is illustrated in Fig. 1, wherein the source of controlled energy in the form of a radiated beam is supplied by an excimer laser 10 having a wavelength of 248 mm and whose optical beam is shown by dashed line 11. The path of beam 11 is directed and controlled by a plurality of optical mirrors 12, 13,14 and 15. Mirror 15 directs beam 11 onto the surface of an implant or substrate 16 to create a microgeometric texturized surface of predetermined design as indicated by the plurality of beams 11a reflected from mirror 15.

In this assemblage, a shutter 17 is positioned at the output of laser 10 to prove a safety interlock as required by the Center for Devices and Radiological Health (CDRH) and to permit the laser to be warmed up and services without engaging the optical beam.

Downstream from shutter 17 and mirror 12, an attenuator 18 is positioned to intercept beam 11 and control the excitation voltage of laser 10. This permits the optical power output of laser 10 to be varied without affecting the optical properties of beam 11. Preferably, attenuator 18 is a variable attenuator which enables the fluence; i.e., energy densities (measured in Joules per square centimeter, J/cm2), impacting the surfaces of the implant or substrate 16 to be varied over a range of about 10 to about 1.

From attenuator 18, laser beam 11 is preferably directed through an homogenizer 19 which serves to increase the uniformity of the intensity of laser beam 11 and maximize its usable fraction; i.e., that portion of laser beam 11 that performs its intended function which, in this instance, is ablation. Homogenizer 19 also serves to form laser beam 11 into a desired geometric shape; e. g., square, rectangular, circular, oval, elliptical, triangular, star-shaped, and the like, before it is passed through an aperture 20 to a mask carousal 21.

As beam 11 is directed through aperture 20 to mask carousal 21, an aperture illuminator 22 is engaged which enables an image having a pre-determined design or pattern to be projected onto the surface of the implant or substrate 16 in visible light before the implant or substrate surface is ablated. By passing the beam 11 through aperture 20, controlling only the desired portion of the pre-selected image projected onto the surface of the implant or substrate 16 can be effected.

Mask carousel 21 is equipped with a plurality of masks, each of which provide pre-selected line and space combinations to be imaged upon the surface of the implant or substrate 16. As the beam 11 exits mask carousel 21, it is directed through an image rotator 23 which turns the beam image being projected from the mask carousel 21 enabling any combination of lines and spaces to be imaged upon the surface of the implant or substrate 16. The image rotator 23 employed in this embodiment is a reflecting version of a Dove prism commonly referred to as a "K mirror". It serves to rotate the image exiting mask carousel 21 about its central axis without bending or distorting its central axis permitting infinite orientation of the exiting image so that a set of predetermined lines can be ablated in any direction.

In the embodiment shown, a TV camera 24, a light source 25 illuminating the surface of the implant or substrate 16 being ablated, a splitter mirror 26 for coaxial illumination, a zoom lens 27 and a projecting lens 28 are provided to enable the projected image pattern and surface of the implant or substrate 16 to be viewed in real time during ablation of the implant or substrate surface. In this embodiment, mirror 14 serves as a combiner and splitter in accepting and reflecting the imaged beam 11 from rotator 23 as well as the illumination from light source 25 and directs them through projecting lens 28 permitting the surface of the implant or substrate to be reflected and directed back through zoom lens 27 to TV camera 24 to accomplish real time ablation viewing; i.e., from about 8 to about 18ns (nanoseconds).

Mirror 15, which directs the imaged beam 11a onto the surface of the implant or substrate 16, is movably mounted by conventional means so that it is capable of rotating and tilting to project the imaged beam 11a in any direction through from about 30 to about 90-relative to the longitudinal axis of the projecting lens 28. Mirror 15 is also mounted so that it can be retracted out of the path of imaged beam 11a enabling imaged beam 11a to be projected directly onto the implant or substrate surface 16. With mirror 15 movably mounted in this manner, an imaged beam can be projected to ablate the inner surfaces of U-shaped implants such as femoral components for knee replacements or the inner and/or outer surfaces of tubular or cylindrical substrates for use in promoting in vitro cell growth.

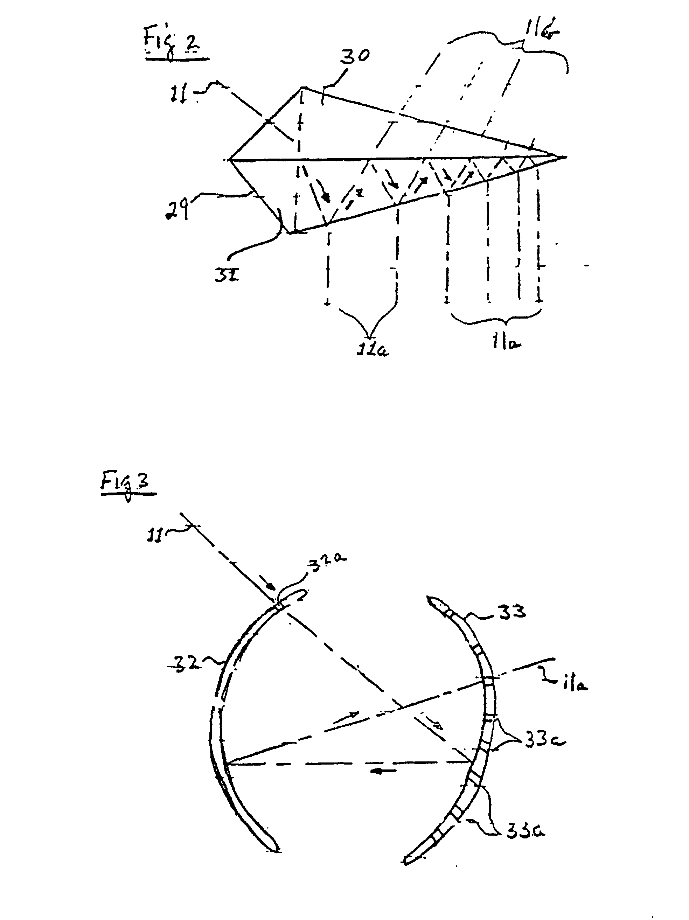

Instead of employing a mask carousel 21 to create pre-determined patterns or designs to be projected onto the surface of an implant or substrate, an optical prism or optical mirrors can be used as illustrated in Figs. 2 and 3.

As shown in Fig. 2, a suitable optical prism 29 has a reflective surface 30 and a refractive surface 31. Prism 29 is positioned to receive optical beam 11 onto reflective surface 30. As optical beam 11 enters the prism 29 through reflective surface 30, it is refracted and reflected a number of times along the length of prism 29 in a path following the general direction of the small arrows. The number of times that beam 11 is refracted and reflected can be predetermined and calculated from the angle, size and index of refraction of the prism or other reflective/refractive means being used. A portion of optical beam 11 passes through refractive surface 31 in the form of a plurality of beams 11a which can either be directly projected onto the surface of an implant or substrate or be directed through an image rotator 23 as described above. A small percentage of optical beams 11a escape through reflective surface 30 shown in long dashed lines as 11b. Prism 29 can be mounted to rotate relative to optical beam 11 and optical beam 11 can also be scanned across surface 30 and/or down its length toward the apex of prism 29 to create any combination of lines and spaces in any direction as desired. Further, although a 3-faced prism is shown, multi-faceted prisms can also be employed to create more complex patterns and designs.

Opposed optical mirrors shown in Fig 3 can also be used to produce pre-determined patterns and designs. In the arrangement shown in Fig. 3, a pair of concave mirrors 32,33 are mounted so that their concave surfaces face each other. The surfaces of mirror 32 are treated to provide a window or port 32a and this mirror is positioned to enable optical beam 11 to pass through window or port 32a. The surfaces of opposed mirror 33 are treated to provide a plurality of windows or ports 33a. After optical beam 11 passes through window or port 32a, it is reflected and refracted a number of times between the opposed surfaces of mirrors 32 and 33 following a path indicated by the short arrows whereupon a portion of the reflected/refracted beams will pass through the plurality of windows or ports 33a in mirror 33. Either or both mirrors 32,33 or optical beam 11 can be moved relative to each other to produce any combination of lines and spaces and thus create an infinite variety of desired patterns and designs,. Gratings can also be used as the refractive/reflective means in place of the optical mirrors.

The assemblage of the system of the invention illustrated in Figs. 1-3 can be readily operated using conventional computer hardware and software. A design data base can be developed for an implant or substrate from which import and export functions can be derived to convert data formats from conventional Computer Aided Design (CAD) programs. Specific microgeometric texturized design patterns can then be prepared and programmed for operation of the assemblage components. Typically, programming of a particular design pattern will be converted into explicit commands for integrated operation of the assemblage components and control of such functions as laser voltage, laser pulse trigger, shutter speed, attenuation, aperture rotation, mask selection, image rotation, mirror tilt and rotation positioning, and the like. For example, software information required to control an optical beam and provide the measurements to texturize a particular implant surface can be generated by a "Digitizing Beam"available from Laser Design, Inc.

Thus, the assemblage and system of the invention permits a plurality of different texturized microgeometric designs or patterns to be concurrently ablated on the surface of an implant or substrate and be provided in pre-designated areas on their surfaces to create separate microgeometric texturized zones. In addition, the system and assemblage of the invention enable ablated implant or substrate texturized patterns having a wide range of dimensions to be obtained such as from about 1 to 2 mm in diameter for dental implants to in excess of 225 mm in length for hip implants.

As mentioned above, the various components comprising the assemblage described in the embodiment of Fig. 1 are commercially available. For example, excimer lasers (10) can be obtained from Lambda Physik, Lumonics, Questek and Rofin Sinar; UV grade fused silica mirrors (12,13,14,15) used for excimer image beam (11) and borosilicate or crown glass used for splitter mirror (26) can be obtained from Action Research, CVI, and Spindler and Hoyer; attenuators (18) can be obtained from Lamson Engineering; homogenizers (19) for specific applications can be obtained from the Laser Technique division of Lambda Physik; aperture illuminators (22) and illuminating light sources (25) can be obtained from Leica, Melles Griot, Nikon, Oriel and Wild; a suitable TV camera (24) can be obtained from Hitachi, Panasonic and Sony; a suitable zoom lens (27) can be obtained from Ealing, Nikon, Oriel and Sony; and a projecting lens (28) can be obtained from Ealing and Newport; optical prisms (29) and optical mirrors (32,33) can be obtained from Rolyn Optic Co. and Reynard Enterprises, Inc.; and gratings can be obtained from Lasiris, Inc. These commercial sources for the various components are obviously not intended to be exhaustive, but are mentioned merely as being representative and illustrative of their commercial availability.

While the invention has been described with particularity and in some detail, it will be appreciated that changes and modifications can be made therein without departing from the scope of the invention recited in the appended claims.

## Claims

1. An implant system for control of cell growth after surgical insertion into a bone (56) or tissue (58,59) of a patient, comprising a region having a first ordered microgeometric, repetitive surface pattern (44,45) in a form of a multiplicity of alternating ridges (45) and grooves (44), each having an established width in a range of about 2 to about 12 microns, and an established depth in a range of about 2 to about 12 micron, each groove (44) having a base "c" and a wall "b", wherein said micro-geometric repetitive pattern defines a guide for a preferential promotion of the rate, orientation and direction of growth of colonies of cells of said bone (56) or tissue (58,59) which are in contact with said surface pattern,
**characterized in that**
each groove defines, in vertical cross-section, an angular relationship of groove base to groove wall that is equal to, or less than ninety degrees.

2. The system as recited in Claim 1,
**characterized in that**
said region comprises an anchor section (53,62) of said implant system.

3. The system as recited in Claim 2,
**characterized in that**
said region further comprises a proximal section (52) comprising a second ordered micro-geometric pattern of ridges (45) and grooves (44) in a range of width of between about 2 and about 12 microns, and a depth in a range of about 2 to 12 microns, said proximal section (52) including a soft tissue adhesion zone (61), and in which said anchor section (53,62) comprises said first pattern of alternating ridges (45) and grooves (44), and constitutes a bone adhesion area.

4. The system as recited in Claim 2,
**characterized in that**
said anchor section (53,62) further comprises a tissue adhesion zone (61) defined by a second ordered micro-geometric repetitive pattern in the form of a multiplicity of alternating ridges (45) and grooves (44) having established widths in a range between about 2 and about 12 microns, and an established depth in a range of about 2 to 12 microns.

5. The system as recited in Claim 2,
**characterized in that**
base materials of said region are selected from a group consisting essentially of the materials of commercially pure titanium, titanium alloys, stainless steel, plastics, ceramics biocompatible glass, and combinations thereof.

6. The system as recited in Claim 2,
**characterized in that**
base materials of said region are selected from a group consisting of essentially the materials of titanium and alloys thereof, stainless steel, ceramics biocompatible glass, and combinations thereof.

7. The system as recited in Claim 1,
**characterized in that**
said repetitive micro-geometric pattern of ridges (45) and grooves (44) of said region comprises orientations which, relative to a planar surface of said system, are selected from a group consisting of vertical, horizontal, diagonal, radial circumferential, and concentric orientations.

8. The system as recited in Claim 7,
**characterized in that**
a surface of said region comprises a coating selected from a group of surfaces consisting of hydroxyapatite, RBM roughening, titanium, plama sprayed, calcium sulfate, biocompatible glass, collagen, growth factor compounds, and combinations thereof.

9. The system as recited in Claim 1,
**characterized in that**
said surface pattern of ridges (45) and grooves (44) defines substantially parallel geometries thereof.

10. The system as recited in Claim 9,
**characterized in that**
said pattern is grid-like.

11. The system as recited in Claim 1,
**characterized in that**
said pattern of ridges (45) and grooves (44) define a radial geometry.

12. The system as recited in Claim 1,
characterized i n that
said pattern of ridges (45) and grooves (44) defines polar geometries.

13. The system as recited in Claim 11,
**characterized in that**
said pattern further includes polar geometries.

14. The system as recited in Claim 5,
**characterized in that**
said repetitive micro-geometric pattern comprises a product of the process selected from the process group consisting of laser etching, acid etching, mechanical etching, and photolithography.

15. The system as recited in Claim 3,
**characterized in that**
said bone (56) includes mandible cortical or maxillofacial bone (56).

16. The system as recited in Claim 2,
**characterized in that**
said anchor comprises a radial longitudinal cross-section selected from the group of geometries consisting of screw-like, tapered screw, cylindrical, tapered cylindrical, buttress thread, and reverse buttress thread.

17. The system as recited in Claim 1,
**characterized in that**
it comprises means for surgical insertion into a maxillofacial bone (56) and tissue (58,59) of a patient, said means comprising a collar section (60) and a distal, anchor-like section (62), said repetitive surface pattern (44,45) being provided on the anchor section (62)

18. The system as recited in Claim 17,
**characterized in that**
said collar section (60) of said implant element also comprises said first repetitive micro-geometric pattern thereupon.

19. The system as recited in Claim 17,
**characterized in that**
said collar section (60) comprises a second ordered micro-geometric pattern of ridges (45) and grooves (44) in a range of width of between about 2 and about 12 microns, and a depth in a range of about 2 to 12 microns.

20. The system as recited in Claim 1,
**characterized in that**
a vertical cross-section of said ridges (45) and grooves (44) comprises a sinusoidal cross-section.

21. The system as recited in Claim 2,
**characterized in that**
a vertical cross-section of said ridges (45) and grooves (44) comprises a sinusoidal cross-section.

22. The system as recited in Claim 17,
**characterized in that**
a vertical cross-section of said ridges (45) and grooves (44) comprises a sinusoidal cross-section.

## Patentansprüche

1. Implantationssystem zur Steuerung eines Zellwachstums nach einem chirurgischen Einfügen in einen Knochen (56) oder einem Gewebe (58,59) eines Patienten, mit einem Bereich, welcher ein mikrogeometrisches, reproduzierbares Oberflächenmuster (44, 45) erster Ordnung in Form einer Vielzahl von sich abwechselnden Erhebungen (45) und Rillen (44), welche jeweils eine bestimmte Breite in einem Bereich von ca. 2 bis ca. 12 Mikron und eine bestimmte Tiefe in einem Bereich von ca. 2 bis ca. 12 Mikron aufweisen, wobei jede Rille (44) einen Boden "c" und eine Wand "b" aufweist, wobei das mikrogeometrische reproduzierbare Muster eine Anleitung für einen bevorzugten Einsatz der Rate, Orientierung und Richtung des Wachstums der Zellkolonien des Knochens (56) oder des Gewebes (58, 59) definiert, welche in Kontakt mit dem Oberflächenmuster sind,
**dadurch gekennzeichnet, dass**
jede Rille in einem vertikalen Querschnitt ein Winkelverhältnis von Rillenboden zu Rillenwand definiert, der kleiner gleich neunzig Grad ist.

2. System nach Anspruch 1,
**dadurch gekennzeichnet, dass**
der Bereich einen Verankerungsabschnitt (53,62) des Implantationssystems aufweist.

3. System nach Anspruch 2,
**dadurch gekennzeichnet, dass**
der Bereich des weiteren einen Rumpfabschnitt (52) aufweist, welcher ein mikrogeometrisches Muster zweiter Ordnung von Erhebungen (45) und Rillen (44) in einem Bereich einer Breite zwischen ca. 2 und ca. 12 Mikron und einer Tiefe in einem Bereich von ca. 2 bis 12 Mikron aufweist, wobei der Rumpfabschnitt (52) eine weiche Gewebeadhäsionszone (61) aufweist und in welchem der Verankerungsabschnitt (53,62) das erste Muster von sich abwechselnden Erhebungen (45) und Rillen (44) aufweist und einen Knochenadhäsionsbereich bildet.

4. System nach Anspruch 2,
**dadurch gekennzeichnet, dass**
der Verankerungsabschnitt (53,62) des weiteren eine Gewebeadhäsionszone (61) aufweist, welche durch ein mikrogeometrisches reproduzierbares Muster in Form von einer Vielzahl von sich abwechselnden Erhebungen (45) und Rillen (44) mit bestimmten Breiten in einem Bereich zwischen ca. 2 und ca. 12 Mikron und einer bestimmten Tiefe in einem Bereich von ca. 2 bis 12 Mikron, definiert ist.

5. System nach Anspruch 2,
**dadurch gekennzeichnet, dass**
Basismaterialien des Bereichs aus einer Gruppe, welche im wesentlichen die Materialien kommerziell reines Titan, Titanlegierungen, rostfreies Stahl, Plastik, keramisches biokompatibles Glas und Kombinationen daraus aufweist, ausgewählt werden.

6. System nach Anspruch 2,
**dadurch gekennzeichnet, dass**
Basismaterialien des Bereichs aus einer Gruppe, welche im wesentlichen die Materialien Titan und Legierungen davon, rostfreies Stahl, Keramik biokompatibles Glas und Kombinationen daraus aufweist, ausgewählt werden.

7. System nach Anspruch 1,
**dadurch gekennzeichnet, dass**
das reproduzierbare mikrogeometrische Muster von Erhebungen (45) und Rillen (44) des Bereichs Orientierungen aufweist, welche relativ zu einer ebenen Oberfläche des Systems aus einer Gruppe, welche verikale, horizontale, diagonale, radiale, umfangsmäßige und konzentrische Ausrichtungen aufweist, ausgewählt werden.

8. System nach Anspruch 7,
**dadurch gekennzeichnet, dass**
eine Oberfläche des Bereichs eine Schicht aufweist, welche aus einer Gruppe von Oberflächen, welche Hydroxyapatit, RBM-Aufrauhung, Titan, gespritztes Plasma, Kalziumsulfat, biokompatibles Glas, Kollagen, Wachstumsfaktormittel und Kombinationen daraus aufweist, ausgewählt werden.

9. System nach Anspruch 1,
**dadurch gekennzeichnet, dass**
das Oberflächenmuster aus Erhebungen (45) und Rillen (44) im wesentlichen parallele Geometrien definiert.

10. System nach Anspruch 9,
**dadurch gekennzeichnet, dass**
das Muster gitterartig ist.

11. System nach Anspruch 1,
**dadurch gekennzeichnet, dass**
das Muster aus Erhebungen (45) und Rillen (44) eine radiale Geometrie definiert.

12. System nach Anspruch 1,
**dadurch gekennzeichnet, dass**
das Muster aus Erhebungen (45) und Rillen (44) polare Geometrien definiert.

13. System nach Anspruch 11,
**dadurch gekennzeichnet, dass**
das Muster des weiteren polare Geometrien aufweist.

14. System nach Anspruch 5,
**dadurch gekennzeichnet, dass**
das reproduzierbare mikrogeometrische Muster ein Produkt eines Prozesses, welcher aus einer Gruppe von Prozessen ausgewählt wurde, welche Laserätzen, Säureätzen, mechanisches Ätzen und Fotolithographie aufweist.

15. System nach Anspruch 3,
**dadurch gekennzeichnet, dass**
der Knochen (56) kortikalen Unterkiefer- oder Gesichtskieferknochen (56) aufweist.

16. System nach Anspruch 2,
**dadurch gekennzeichnet, dass**
die Verankerung einen radialen Längsschnitt aufweist, welcher aus einer Gruppe von Geometrien ausgewählt ist, die schraubenartig, zugespitzte Schraube, zylindrisch, zugespitzt zylindrisch, mit Sägezahngewinde und mit umgekehrtem Sägezahngewinde aufweist.

17. System nach Anspruch 1,
**dadurch gekennzeichnet, dass**
es Mittel für das chirurgische Einfügen in einen Gesichtskieferknochen (56) und Gewebe (58,59) eines Patienten aufweist, wobei die Mittel einen Ringabschnitt (60) und einen distalen, verankerungsähnlichen Abschnitt (62) aufweisen, wobei das reproduzierbare Muster (44,45) mit einem Verankerungsabschnitt (62) versehen ist.

18. System nach Anspruch 17,
**dadurch gekennzeichnet, dass**
der Ringabschnitt (60) des Implantationselements des weiteren ein erstes reproduzierbares mikrogeometrisches Muster darauf aufweist.

19. System nach Anspruch 17,
**dadurch gekennzeichnet, dass**
der Ringabschnitt (60) ein zweites mikrogeometrisches Muster aus Erhebungen (45) und Rillen (44) in einem Bereich einer Breite zwischen ca. 2 und ca. 12 Mikron und einer Tiefe in einem Bereich von ca. 2 bis ca. 12 Mikron aufweist.

20. System nach Anspruch 1,
**dadurch gekennzeichnet, dass**
ein vertikaler Querschnitt der Erhebungen (45) und der Rillen (44) einen sinusförmigen Querschnitt aufweist.

21. System nach Anspruch 2,
**dadurch gekennzeichnet, dass**
ein vertikaler Querschnitt der Erhebungen (45) und der Rillen (44) einen sinusförmigen Querschnitt aufweist.

22. System nach Anspruch 17,
**dadurch gekennzeichnet, dass**
ein vertikaler Querschnitt der Erhebungen (45) und Rillen (44) einen sinusförmigen Querschnitt aufweist.

## Revendications

1. Système d'implant pour le contrôle d'une croissance cellulaire après une insertion chirurgicale dans un os (56) ou un tissu (58, 59) d'un patient, comportant une partie ayant un premier motif de surface répétitif microgéométrique ordonné (44, 45) sous la forme d'une multiplicité d'arêtes (45) et de rainures (44) alternées, ayant chacune une largeur bien déterminée dans une gamme d'environ 2 à environ 12 microns et une profondeur déterminée dans une gamme d'environ 2 à environ 12 microns, chaque rainure (44) ayant une base « c » et une paroi « b », dans lequel ledit motif répétitif microgéométrique définit un guide pour un avancement préférentiel du taux, de l'orientation et de la direction de croissance des colonies de cellules dudit os (56) ou tissu (58, 59) qui sont en contact avec ledit motif de surface,
**caractérisé en ce que**
chaque rainure définit, en section droite verticale, une relation angulaire entre la base de la rainure et la paroi de la rainure qui est égale, ou inférieure, à quatre-vingt dix degrés.

2. Système tel qu'exposé dans la revendication 1,
**caractérisé en ce que**
ladite partie comporte une portion d'ancrage (53, 62) dudit système d'implant.

3. Système tel qu'exposé dans la revendication 2,
**caractérisé en ce que**
ladite partie comporte en outre une portion proximale (52) comportant un second motif microgéométrique ordonné de crêtes (45) et de rainures (44) dans une gamme de profondeur entre environ 2 et environ 12 microns, et une profondeur dans une gamme d'environ 2 à 12 microns, ladite portion proximale (52) comprenant une zone d'adhérence de tissu mou (61), et dans lequel ladite portion d'ancrage (53, 62) comporte ledit premier motif de rainures (44) et d'arêtes (45) alternées, et constitue une surface d'adhérence à l'os.

4. Système tel qu'exposé dans la revendication 2,
**caractérisé en ce que**
ladite portion d'ancrage (53, 62) comporte en outre une zone d'adhérence au tissu (61) définie par un second motif répétitif microgéométrique ordonné sous la forme d'une multiplicité de crêtes (45) et de rainures (44) alternées ayant des largeurs déterminées dans une gamme entre environ 2 et environ 12 microns, et une profondeur déterminée dans une gamme d'environ 2 à 12 microns.

5. Système tel qu'exposé dans la revendication 2,
**caractérisé en ce que**
les matériaux de base de ladite partie sont choisis à partir d'un groupe se composant essentiellement de matériaux en titane pur commercialement, d'alliages de titane, d'acier inoxydable, de plastiques, de verre biocompatible avec des céramiques et de combinaisons de ceux-ci.

6. Système tel qu'exposé dans la revendication 2,
**caractérisé en ce que**
les matériaux de base de ladite partie sont choisis à partir d'un groupe se composant essentiellement de matériaux en titane et alliages de titane, d'acier inoxydable, de verre biocompatible avec des céramiques et de combinaisons de ceux-ci.

7. Système tel qu'exposé dans la revendication 1,
**caractérisé en ce que**
ledit motif microgéométrique répétitif d'arêtes (45) et de rainures (44) de ladite partie comporte des orientations qui, par rapport à une surface plane dudit système, sont choisies à partir d'un groupe se composant d'orientations verticales, horizontales, diagonales, circonférentielles radiales, et concentriques.

8. Système tel qu'exposé dans la revendication 7,
**caractérisé en ce que**
une surface de ladite partie comporte un revêtement choisi à partir d'un groupe de surfaces se composant d'hydroxy-apatite, de RBM (cellules d'os de rat) rendant rugueux, de titane, de plasma pulvérisé, de sulfate de calcium, de verre biocompatible, de collagène, de composés de facteurs de croissance, et de combinaisons de ceux-ci.

9. Système tel qu'exposé dans la revendication 1,
**caractérisé en ce que**
ledit motif de surface d'arêtes (45) et de rainures (44) définit des géométries sensiblement parallèles.

10. Système tel qu'exposé dans la revendication 9,
**caractérisé en ce que**
ledit motif forme une grille.

11. Système tel qu'exposé dans la revendication 1,
**caractérisé en ce que**
ledit motif d'arêtes (45) et de rainures (44) définit une géométrie radiale.

12. Système tel qu'exposé dans la revendication 1,
**caractérisé en ce que**
ledit motif d'arêtes (45) et de rainures (44) définit des géométries polaires.

13. Système tel qu'exposé dans la revendication 11,
**caractérisé en ce que**
ledit motif comprend en outre des géométries polaires.

14. Système tel qu'exposé dans la revendication 5,
**caractérisé en ce que**
ledit motif microgéométrique répétitif comporte un produit d'un procédé choisi parmi le groupe de procédés se composant de la gravure au laser, la gravure à l'acide, la gravure mécanique, et la photolithographie.

15. Système tel qu'exposé dans la revendication 3,
**caractérisé en ce que**
ledit os (56) comprend un os maxillo-facial ou cortical mandibulaire (56).

16. Système tel qu'exposé dans la revendication 2,
**caractérisé en ce que**
ledit ancrage comporte une section droite longitudinale radiale choisie parmi le groupe de géométries se composant d'hélice, d'hélice conique, cylindrique, cylindrique conique, de filetage arqué, et de filetage arqué inverse.

17. Système tel qu'exposé dans la revendication 1,
**caractérisé en ce que**
il comporte des moyens pour une insertion chirurgicale dans un os maxillo-facial (56) et un tissu (58, 59) d'un patient, lesdits moyens comportant une portion col (60) et une portion d'ancrage (62), ledit motif de surface répétitif (44, 45) étant ménagé sur la portion d'ancrage (62).

18. Système tel qu'exposé dans la revendication 17,
**caractérisé en ce que**
ladite portion col (60) dudit élément d'implant comporte également ledit premier motif microgéométrique répétitif.

19. Système tel qu'exposé dans la revendication 17,
**caractérisé en ce que**
ladite portion col (60) comporte un second motif microgéométrique ordonné d'arêtes (45) et de rainures (44) dans la gamme de largeur entre environ 2 et environ 12 microns, et une profondeur dans une gamme d'environ 2 à 12 microns.

20. Système tel qu'exposé dans la revendication 1,
**caractérisé en ce que**
une section droite verticale desdites arêtes (45) et rainures (44) comporte une section droite sinusoïdale.

21. Système tel qu'exposé dans la revendication 2,
**caractérisé en ce que**
une section droite verticale desdites arêtes (45) et rainures (44) comporte une section droite sinusoïdale.

22. Système tel qu'exposé dans la revendication 17,
**caractérisé en ce que**
une section droite verticale desdites arêtes (45) et rainures (44) comporte une section droite sinusoïdale.
